# EUROPEAN PATENT SPECIFICATION

(11) **EP 3 468 494 B1**
(45) Date of publication and mention of the grant of the patent: **21.07.2021**
(21) Application number: 17733989.2
(22) Date of filing: 12.06.2017
(51) Int. Cl.: A61B 1/273, A61B 1/307, A61B 1/31, A61N 1/32, A61B 18/14, A61B 18/00, A61B 1/00, A61B 1/267

(54) **ADD-ON CAP AND SYSTEM FOR ELECTROPORATION**
ZUSATZKAPPE UND SYSTEM ZUR ELEKTROPORATION
CAPUCHON COMPLÉMENTAIRE ET SYSTÈME D'ÉLECTROPORATION

(30) Priority: 14.06.2016 DK 201670426
(43) Date of publication of application: 17.04.2019
(73) Proprietor: Aarhus Universitet, 8000 Aarhus C (DK); Region Midtjylland, 8800 Viborg (DK)
(72) Inventor: ØRNTOFT, Mai-Britt, Worm, 8350 Hundslund (DK); GEERT, Torben, 8960 Randers SØ (DK); LYSKJÆR, Iben, 8870 Langå (DK); CALLESEN, Morten, Møbjerg, 2920 Charlottenlund (DK); UHRE, Vibeke, 8380 Trige (DK); RASMUSSEN, Mads Kristoffer, Heilskov, 8220 Brabrand (DK)
(74) Representative: Weldon O'Brien Ltd.
(86) International application number: PCT/EP2017/064280
(87) International publication number: WO 2017/216102

(56) References cited:
- US-A- 6 086 583
- US-A1- 2005 080 411
- US-A1- 2007 179 535
- US-A1- 2012 172 784
- US-A1- 2014 012 247
- US-A1- 2015 073 409
- US-A1- 2016 058 464

## Description

The present disclosure relates to an add-on cap for an instrument, such as an endoscope, for electroporation of tissue. The disclosure further relates to a system for electroporation of tissue, comprising the add-on cap, and to a method for electroporation of tissue. The method is not part of the invention.

### Background of invention

Electroporation is a medical and molecular biology technique in which an electric field is applied to cells or molecules in order to increase the permeability of the cell membrane, allowing chemicals, drugs, or DNA to be introduced into the cell. Electroporation has a number of possible fields of application, and can be used both reversibly and irreversibly. In a typical electroporation process, short and intense electric pulses are generated to transiently permeabilize the cell membrane.

In reversible electroporation, which may be used in relation to for example chemotherapy, the electric field is below an electric field threshold and allows the cell to repair after the treatment. Reversible electroporation may involve getting a substance, such as a drug or gene, into a cell or molecule that is normally not permeable for this substance without inducing cell death. The electric field threshold for a cell is individual for the cell. In irreversible electroporation the electric field is greater than the electric field threshold, which creates permanent nanopores in the cell membrane disrupting the cellular homeostasis. As a consequence the cell dies by apoptosis.

Reversible electroporation is known and performed both *in vitro* and *in vivo. In vivo* electroporation is typically performed using needle electrodes that are inserted into for example a tumor and surrounding tissue. One device for reversible electroporation is the Cliniporator system. Irreversible electroporation may also be performed *in vivo.* One device for irreversible electroporation is the NanoKnife system which also uses needle or spear like electrodes.

WO 2011/030322 describes an apparatus for carrying out research experiments and prophylactic or treatment procedures on tissue comprising a chamber for receiving tissue and a receiver for receiving an instrument such as an endoscope. Vacuum draws tissue into the chamber, in which electroporation is applied by means of electrodes.

### Summary of invention

The present disclosure relates to an improved add-on cap for electroporation of tissue comprising: at least one closed sidewall, such as a cylindrical sidewall; a proximal open end; a distal open end; the at least one closed sidewall and the proximal and distal open ends defining a hollow body having an internal volume; and two electrodes on opposite sides of the internal volume, the electrodes configured to apply an electric field for electroporation of tissue inside the internal volume, wherein the proximal open end is adapted to be sealingly engaged to a tip of an instrument, such as an endoscope.

Since the cap with two internal electrodes has two open ends, of which one can be sealingly engaged with the tip of the instrument, the cap can easily be attached and used with an endoscope to perform electroporation, while providing visibility both when positioning the device before the electroporation and during the electroporation by imaging tissue that is aspirated into the cap from the inside of the cap. If a vacuum generator is connected to the internal volume through the endoscope, the tissue can be treated inside the cap without the need for needle electrodes while the process is observed from the inside of the cap.

The presently disclosed add-on cap can be attached directly to the tip of an endoscope. The term endoscope is to be construed as any endoscope, including rigid and flexible endoscopes. Preferably the at least one closed sidewall and the open ends of the cap form a combined receiver for the instrument and internal volume for electroporation of tissue, such that the add-on cap extends in an axial direction of the instrument. The cap may therefore have a substantially cylindrical shape with two open ends, wherein the proximal open end fits an endoscope. Electric wiring to the electrodes may be provided by means of cables on the outside of the closed sidewall, wherein the cables are connected to the electrodes through holes in the closed sidewall. By arranging the cables on the outside, the cap with cables can be easily attached to the tip of the endoscope by threading the proximal open end over the tip. The cables can then run along the endoscope on the outside of the endoscope and can for example be clipped onto the endoscope. This setup further improves both the visibility inside the internal volume and the possibilities of additional (action) inside the internal volume, such as biopsy and/or chemotherapy by injection, during electroporation.

The present disclosure further relates to a system for electroporation of tissue comprising: an endoscope; the presently disclosed add-on cap adapted to be mounted on the tip of the endoscope and extending in an axial direction of the endoscope; and an electric current generator.

The present disclosure further relates to a method for electroporation of tissue using the presently disclosed system, wherein the add-on cap is mounted on the tip of the endoscope, comprising the steps of: positioning the distal open end of the add-on cap adjacent to tissue to be treated with electroporation, wherein the positioning is visually assisted by imaging through the add-on cap from the distal open end to the proximal open end; applying vacuum in the internal volume through the proximal open end of the add-on cap, thereby aspirating tissue into the internal volume; and electroporating the aspirated tissue by applying an electric field between the electrodes of the add-on cap. The imaging can be provided through a normal lens of the endoscope. In one embodiment the method comprises the steps of: before electroporation of the aspirated tissue, measuring the impedance between the two electrodes and comparing against a predetermined impedance threshold; and, based on the comparison, determine if electroporation is to be started. The combination of visual observation of the cap throughout the process of positioning the cap and aspiration of tissue and measuring the impedance between the electrodes provides an efficient way of ensuring that the electroporation is performed correctly. Measuring the impedance may provide information about the contact between the electrodes and the aspirated tissue, and about the characteristics of the material, which may be further used to inform the user of a result of a treatment. Devices for the treatment of tissue by electroporation are known e.g. from US 2007/179535 A1 and US2014/012247 A1. Further relevant endoscopic caps are disclosed in US 6,086,583 A1.

### Description of drawings

**Fig. 1** shows a first embodiment of the presently disclosed add-on cap for electroporation.
**Fig. 2** shows the add-on cap of fig. 1 from a different angle.
**Fig. 3** shows the add-on cap of figs. 1-2 from a different angle.
**Fig. 4** shows a second embodiment of the presently disclosed add-on cap for electroporation.
**Fig. 5** shows the add-on cap of fig. 4 mounted on the tip of a gastroscope.
**Fig. 6** shows a view from the inside of the presently disclosed add-on cap for electroporation, the cap being position in the bowel cavity.
**Fig. 7** shows a view from the inside of the presently disclosed add-on cap for electroporation, wherein intestinal mucosa is aspirated into the cap.
**Fig. 8** shows a view from the inside of the presently disclosed add-on cap for electroporation, wherein intestinal mucosa is aspirated into the cap and a sclerotherapy needle is used to inject a substance into the aspirated tissue.
**Fig. 9** shows a view from the inside of the presently disclosed add-on cap for electroporation, wherein a biopsy forceps is used to collect a sample of the intestinal mucosa.

### Detailed description of the invention

The present disclosure relates to a cap for electroporation of tissue comprising:
- at least one closed sidewall, such as a cylindrical sidewall; a proximal open end; a distal open end; the at least one closed sidewall and the proximal and distal open ends defining a hollow body having an internal volume;
- two electrodes on opposite sides of the internal volume, the electrodes configured to apply an electric field for electroporation of tissue inside the internal volume,
wherein the proximal open end is adapted to be sealingly engaged to a tip of an instrument, such as an endoscope.

The terms 'proximal' and 'distal' in the present disclosure are defined in relation to the instrument i.e. the proximal end of the cap is located towards the instrument and the distal end of the cap is located towards the tissue.

The fact that the cap has two open ends, of which one can be sealingly engaged with the tip of the instrument such as an endoscope, makes it possible to attach it to the tip of the scope such that an electroporation process can be performed while the user has visibility through the cap. The prior art device disclosed in WO 2011/030322 has one receiver for receiving an instrument and one chamber, wherein the receiver and chamber are radially offset. In the presently disclosed cap, a closed sidewall forms a hollow body with two open ends, of which one is directly attached around the tip of the instrument. Thereby, the user will have visibility throughout an electroporation process. In a first phase, during positioning of the cap, the user will have visibility through the cap, which forms and extension of the scope. In contrast to the device in WO 2011/030322, the user is able to see the tissue in front of the cap. The visibility can be provided by means of standard imaging capabilities of e.g. an endoscope. In a second phase tissue is drawn into the cap by means of a vacuum generator connected through the endoscope. In the second phase the user is able to observe the tissue being drawn into the cap from the inside of the cap. In a third phase electroporation is performed by applying an electric field through the electrodes in the hollow body. The add-on cap can therefore be mounted directly on the tip of an instrument and used without being further adapted to an existing system. Preferably, the cap is provided with cables connected to the electrodes. In one embodiment the cables are mounted on the outside on the closed sidewall and connected to the electrodes through holes in the closed sidewall. By arranging the cables on the outside, the cap with cables can be easily attached to the tip of the endoscope by threading the proximal open end over the tip. The cables can then run along the endoscope on the outside of the endoscope and can for example be clipped onto the endoscope. The presently disclosed add-on cap may therefore be seen as a device capable of providing electroporation to an endoscope without need for further integration, while allowing visual observation of both the positioning and the actual electroporation through the cap.

Preferably, the at least one closed sidewall and the two open ends of the cap form a combined receiver for the instrument and internal volume for electroporation of tissue, such that the add-on cap extends in an axial direction of the instrument. In one embodiment the longitudinal axis of the add-on cap is an extension of the longitudinal axis of the instrument when the cap is attached to the instrument. In this configuration there is direct visibility from the proximal open end to the distal open end. The sidewall may be formed as an extension of the instrument. The distal open end may be an opening substantially perpendicular to an axial extension of the instrument.

'Vacuum' may generally be referred to a pressure much less than atmospheric pressure. In relation to the present invention, 'vacuum' is construed broadly as an at least partial vacuum (imperfect vacuum) with the intention that the vacuum is capable of drawing tissue into the cap. In one embodiment the proximal open end is sealingly engaged to the instrument such that vacuum can be applied in the internal volume through the proximal open end. In this regard the internal volume of the cap may be seen as a closed volume during electroporation since the proximal end is sealingly engaged to the tip of the instrument and the distal end open end is typically sealed by tissue that is drawn into the cap. The internal volume may therefore be substantially sealed from both open ends when vacuum is applied through the proximal open end and tissue is drawn into the internal volume through the distal open end.

There are several options and scenarios for using the cap for electroporation. One option is that a substance, such as a drug, is added to the circulatory system. During the electroporation process the permeability of the cell membrane is increased, which allows the substance in the circulatory system to be translocated into the cell. A second option is to fill the internal volume with a liquid comprising chemicals, drugs, a gene therapy vector, siRNA, or DNA to be introduced into the cell through the proximal open end while tissue is inside the cap and blocks the distal open end of the cap. Adenoviral CRISPR-Cas vector delivery may also be done using this option. During this process it is possible to observe the treatment from the scope from the inside of the cap. A third option, which is possible with the presently disclosed add-on cap for electroporation, is to use an injection needle through the add-on cap to inject a substance in the tissue. Also in this scenario it is an advantage that the injection can be observed visually through the cap.

### Physical properties of the cap

The cap may be dimensioned to fit a number of instruments, such as an endoscope, including gastroscopes, colonoscopes, cystoscopes, nephroscopes, bronchoscopes, sigmoideumscopes and borescopes. The diameter of the cap, in particular the diameter of the sidewall, may be for example 1-20 mm, or 5-20 mm, or 10-20 mm, or 5-15 mm, or 5-10 mm. In one embodiment, suitable for gastroscopes for use in the stomach and duodenum, the cap has an outer diameter of 8.8 mm. In another embodiment, suitable for colonoscopes for use in the large intestine, the cap has an outer diameter of 13.2 mm. In one embodiment, suitable for flexible cystoscopes for use in the urinary bladder and ureter, the cap has an outer diameter of 2.7 mm. Other possible and known outer diameters of caps are 13.9 mm and 14.9 mm.

The length of the cap, i.e. the distance between the two open ends, may be for example in the range of 1-20 mm, or 5-20 mm, or 10-20 mm, or 5-15 mm, or 5-10 mm. A suitable length can be said to depend on the diameter of the sidewall and also has to take into account the visibility through the cap from the inside of the cap. A longer cap will have less visibility outside the cap than a shorter. On the other hand, a longer cap has a larger internal volume, which may be useful for example for treating more tissue. Therefore a suitable combination of diameter and length may be a diameter between 5 and 15 mm and a length between 5 and 15 mm.

Preferably, the proximal end of the add-on cap fits an exterior surface of a distal end of the instrument. In this regard the at least one closed sidewall may be shaped such that it can be snapped or threaded onto the tip of the instrument, which typically has a circular cross-section. The open proximal end may therefore be substantially circular and the at least one closed sidewall may be a substantially cylindrical closed surface. The proximal and distal open ends may then be represented by a top and bottom part of a cylinder. The at least one closed sidewall and the open ends may form an endoscopic cap. The hollow body, or at least a proximal section of the closed sidewall, may be tapered and/or conical and/or funnel shaped in order to be sealingly attached around the tip of the instrument. The closed sidewall may furthermore be seen as an extension of the instrument in an axial direction of the instrument when the cap is mounted on the instrument.

Although there are two electrodes on opposite sides of the internal volume on the inside of the closed sidewall it may be possible in some embodiments to further increase the visibility by using a transparent cap. The presently disclosed add-on cap may be therefore be a transparent endoscopic cap, such as a hollow transparent cylinder. The add-on cap may be made of a plastic material and/or silicone and may have a smooth exterior surface to avoid that tissue is damaged.

Other shapes and cross-sections of the cap are also possible. For example the hollow body may have a substantially rectangular three-dimensional shape. A cross section of the hollow body may also be elliptical or form a polygon, preferably an equilateral and/or convex polygon, a polygon such as a triangle, square, pentagon, hexagon, or a heptagon.

### Temperature sensor(s), impedance measurement, electric properties

The presently disclosed add-on cap for electroporation of tissue may further comprise at least one temperature sensor in the internal volume. Preferably, the at least one temperature sensor is mounted on the inside of the at least one closed sidewall between the two electrodes. Generated heat is a factor to take into account for *in vivo* electroporation. If too much heat is dissipated, surrounding tissue in the body may be damaged. The fact that the add-on cap may provide a closed internal volume during electroporation when tissue is drawn into the cap protects surrounding tissue during the process. A temperature sensor may be placed on the inside of the at least one closed sidewall between the two electrodes where it does not interfere with the process and the visibility through the cap. A temperature sensor may keep the user informed whether there is any risk of damage of surrounding tissue throughout the electroporation process. If the measured temperature is above a predetermined temperature threshold, the electroporation can be stopped manually or automatically.

In one embodiment two temperature sensors are mounted on opposite sides of the internal volume between the two electrodes. Two sensors on opposite sides provide additional security by avoiding that only the temperature on one side is measured and the temperature is accidently higher on the other side.

In a further embodiment of the add-on cap the two electrodes are configured to measure an impedance between the two electrodes. The two electrodes may be planar electrodes mounted on the inside of the at least one closed sidewall. Impedance is defined as the frequency domain ratio of the voltage to the current. Impedance can be said to be an AC property which cannot be measured as easily as a resistance. Measurements of impedance may be carried out at one frequency, or the variation of device impedance over a range of frequencies may be of interest. For the present add-on cap the impedance may for example be carried out a measurement frequency of approximately 50 kHz.

The electroporation process may be used both for reversible and irreversible electroporation. The applied voltage is not limited to any voltage range but may be between 30 and 400 V. The applied pulses may have a length of 10 µs to 1000 µs, more preferable between 200 µs and 800 µs, even more preferable between 500 µs and 700 µs, such as 600 µs. The time between the pulses may be 10 ms to 1000 ms, preferably between 10 ms and 500 ms, more preferably between 10 and 200 ms.

### System for electroporation of tissue

The present disclosure further relates to a system for electroporation of tissue comprising:
- an endoscope;
- an add-on cap as described above, the add-on cap adapted to be mounted on the tip of the endoscope and extending in an axial direction of the endoscope; and
- an electric current generator.

The endoscope may be a gastroscope, or a colonoscope, or a cystoscope, or a nephroscope, or a bronchoscope, or a borescope. The system may further describe a vacuum generator in connection with the internal volume through the proximal open end of the add-on cap. Endoscopes typically have internal channels which can be used to apply a vacuum.

The system may further comprise means (a device) for measuring an impedance. The system may further comprise processing means and/or a processing module. The processing module could be a computer or part of a computer. The processing module may be configured to measure and process the impedance between the two electrodes. The impedance can then be compared against a predefined threshold in order to determine if electroporation can be started. The impedance check can be automatic and and/or may also involve an indication to the user whether the measured impedance is above or below the threshold.

The processing module may also be configured to compare a temperature measured by the temperature sensor(s) against a predetermined temperature threshold.

The add-on cap and system may comprise a pair of cables. The cables connected to the electrodes may be positioned on the outside of the endoscope along the endoscope. By arranging the cables on the outside, the cap with cables can be easily attached to the tip of the endoscope by threading the proximal open end over the tip. The cables can then run along the endoscope on the outside of the endoscope and can for example be clipped onto the endoscope.

The system may further comprise a biopsy forceps for taking a sample from the tissue and/or a hypodermic needle for injecting a substance into the tissue. The presently disclosed add-on cap enables visibility inside the internal volume during electroporation.

### Method for electroporation of tissue

The present disclosure further relates to a method for electroporation of tissue using the system according to the present disclosure, wherein the add-on cap is mounted on the tip of the endoscope, the method comprising the steps of:
a) positioning the distal open end of the add-on cap adjacent to tissue to be treated with electroporation, wherein the positioning is visually assisted by imaging through the add-on cap from the distal open end to the proximal open end;
b) applying vacuum in the internal volume through the proximal open end of the add-on cap, thereby aspirating tissue into the internal volume; and
c) electroporating the aspirated tissue by applying an electric field between the electrodes of the add-on cap.

The method may use any variant of the presently disclosed add-on cap and system. The steps are sequential. One advantage in relation to the devices and methods that are known in the art is that the whole process may be visually assisted by an imaging modality from the inside of the cap.

Since the system may comprise a processing module configured to measure and process the impedance between the two electrodes, it is possible to measure the impedance and compare against a predefined threshold to verify that the electrodes have good contact with the tissue before the electroporation starts. In one embodiment electroporation is started if the measured impedance is below the predefined impedance threshold. The method may further comprise the steps of:
i. before electroporation of the aspirated tissue, measuring the impedance between the two electrodes and comparing against a predetermined impedance threshold;
ii. based on the comparison, determine if electroporation is to be started.
Steps i) and ii) are preferably performed sequentially between steps b) and c).
If the measured impedance is above a predefined impedance threshold it may show that the contact between the electrodes and tissue is not satisfactory. In this case the distal open end may be repositioned to have a better position or angle. In one embodiment the steps a-b and i-ii may be iteratively repeated until the tissue is arranged such that the measured impedance is below the predefined impedance threshold.

The method may further comprise the step of measuring the temperature inside or outside the add-on cap during electroporation. If the temperature exceeds a predefined temperature threshold the electroporation may be stopped or interrupted in order not to damage surrounding tissue (step c).

### Detailed description of drawings

The invention will in the following be described in greater detail with reference to the accompanying drawings. The drawings are exemplary and are intended to illustrate some of the features of the presently disclosed add-on cap and system for electroporation, and are not to be construed as limiting to the presently disclosed invention.

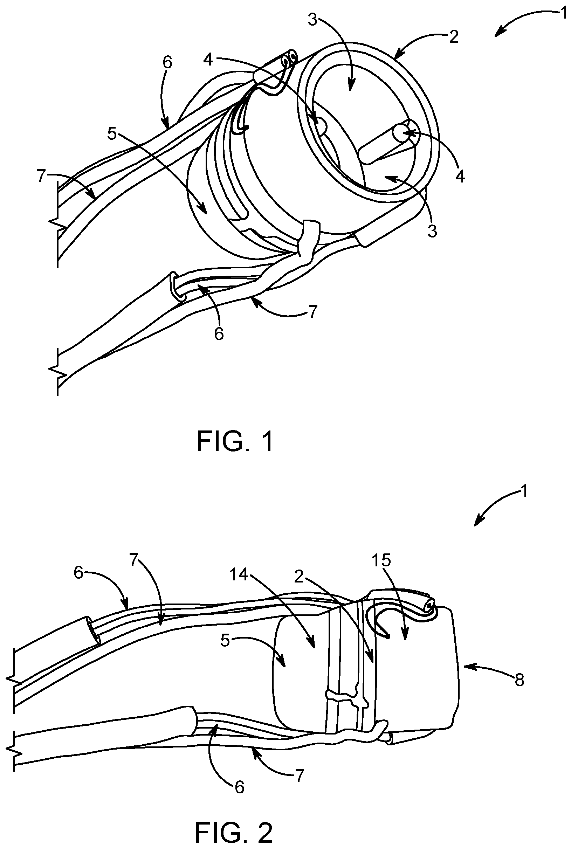

Fig. 1 shows a first embodiment of the presently disclosed add-on cap (1) for electroporation. The cap has a closed sidewall (2) two electrodes (3) on opposite sides of the internal volume. In the example there are two temperature sensors (4) on opposite sides of the internal volume between the two electrodes (3). A proximal open end (5) is designed to be sealingly engaged to fit the tip of an endoscope. A first pair of cables (6) are connected to the temperature sensors (4) and a second pair of cables (7) are connected to the electrodes (3).

Fig. 2 shows the add-on cap (1) of fig. 1 from a different angle. It can be seen that a proximal section (14) of the closed sidewall (2) is slightly tapered to fit the tip of an endoscope. The cap also has a distal section (15) which may be transparent. Tissue may be drawn into the cap through the distal open end (8).

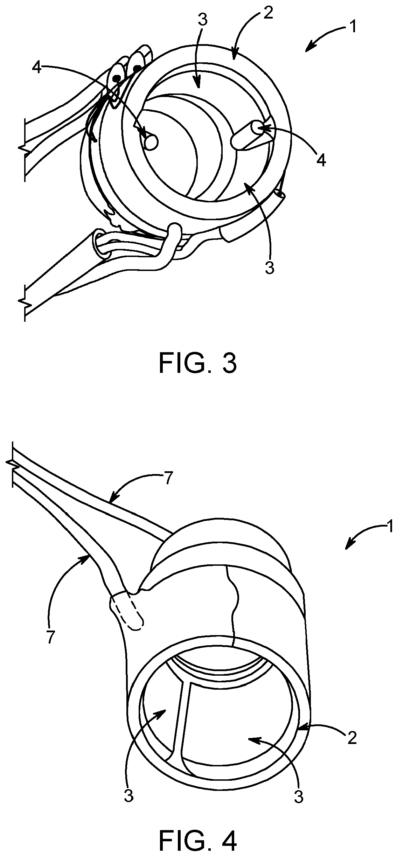

Fig. 3 shows the add-on cap (1) of figs. 1-2 from a different angle. The closed sidewall (2) has a substantially circular cross-section.

Fig. 4 shows a second embodiment of the presently disclosed add-on cap (1) for electroporation. This embodiment does not have any temperature sensors. The sidewall (2) is substantially cylindrical.

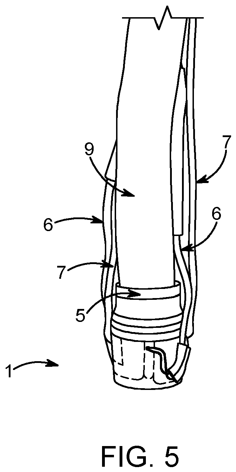

Fig. 5 shows the add-on cap (1) of fig. 4 mounted on the tip of a gastroscope (9). It can be noted that the first pair of cables (6) connected to the temperature sensors (4) and the second pair of cables (7) connected to the electrodes (3) are placed outside the gastroscope (9).

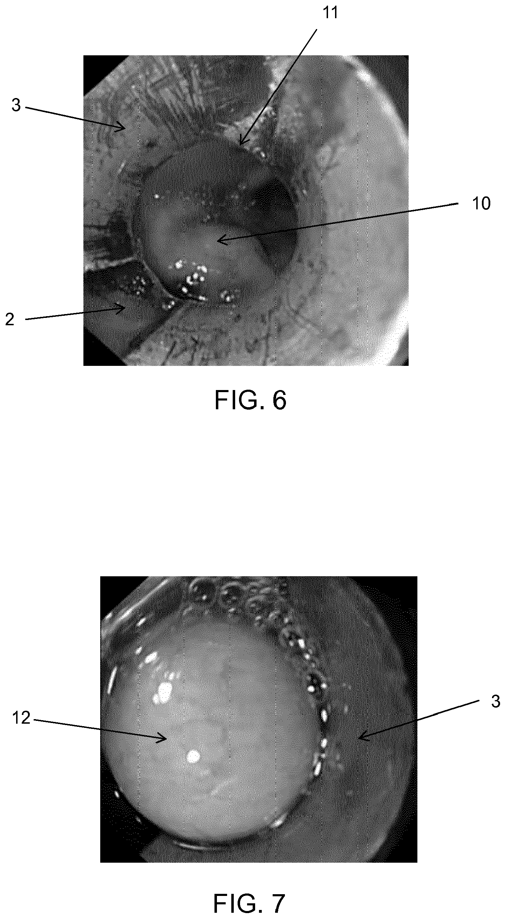

Fig. 6 shows a view from the inside of the presently disclosed add-on cap (1) for electroporation, the cap being position in the bowel cavity (10). The cap has a closed sidewall (2) and two electrodes (3) on opposite sides of the internal volume. This is a typical view during positioning of the device. The distance between the rim (11) and the lens of the imaging in the endoscope determines the angles in which visibility is provided during positioning of the device.

Fig. 7 shows a view from the inside of the presently disclosed add-on cap (1) for electroporation, wherein intestinal mucosa (12) is aspirated into the cap. It can be noted that the distal open end (8) is thereby substantially sealed.

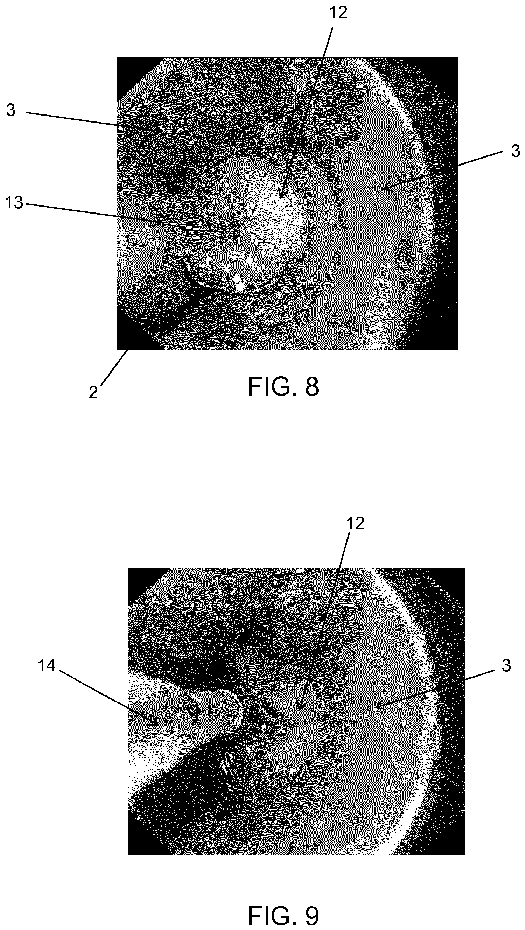

Fig. 8 shows a view from the inside of the presently disclosed add-on cap (1) for electroporation, wherein intestinal mucosa (12) is aspirated into the cap and a sclerotherapy needle (13) is used to inject a substance into the aspirated tissue. The process is observed from the inside of the cap (1).

Fig. 9 shows a view from the inside of the presently disclosed add-on cap (1) for electroporation, wherein a biopsy forceps (14) is used to collect a sample of the intestinal mucosa (12). The process is assisted by imaging from the inside of the cap (1).

## Claims

1. An add-on cap (1) for electroporation of tissue comprising:
- at least one closed sidewall (2), such as a cylindrical sidewall; a proximal open end (5); a distal open end (8); the at least one closed sidewall and the proximal and distal open ends defining a hollow body having an internal volume;
- two electrodes (3) on opposite sides of the internal volume, the electrodes configured to apply an electric field for electroporation of tissue inside the internal volume,
**characterised in that** the proximal open end (5) is adapted to be sealingly engaged to a tip of an instrument, such as an endoscope and **in that** the at least one closed sidewall (2) and the open ends form a combined receiver for the instrument and internal volume for electroporation of tissue.

2. The add-on cap according to claim 1, wherein the add-on cap is adapted to be mounted on the tip of an oblong instrument, such as an endoscope, the add-on cap extending in an axial direction of the instrument.

3. The add-on cap according to claim 2, wherein a longitudinal axis of the add-on cap is an extension of a longitudinal axis of the instrument.

4. The add-on cap according to claim 2 or 3, wherein the add-on cap provides direct visibility from the proximal open end (5) to the distal open end (8).

5. The add-on cap according to any of claims 2 to 4, wherein the distal open end (8) is an opening substantially perpendicular to an axial extension of the instrument.

6. The add-on cap according to any of the preceding claims, wherein the two electrodes (3) are configured to measure an impedance between the two electrodes.

7. The add-on cap according to any of the preceding claims, wherein the two electrodes (3) are planar electrodes mounted on the inside of the at least one closed sidewall (2).

8. The add-on cap according to any of the preceding claims, further comprising at least one temperature sensor (4), optionally the at least one temperature sensor (4) is mounted on the inside of the at least one closed sidewall between the two electrodes, optionally two temperature sensors (4) are mounted on opposite sides of the internal volume between the two electrodes.

9. The add-on cap according to any of the preceding claims, further comprising cables (7) connected to the two electrodes (3), optionally the cables (7) are mounted outside the closed sidewall and connect to the electrodes through holes in the closed sidewall.

10. The add-on cap according to any of the preceding claims, wherein the proximal open end (5) is sealingly engaged to the instrument such that vacuum can be applied in the internal volume through the proximal open end, optionally tissue to be treated with electroporation is drawn into the internal volume through the distal open end (8) by the generated vacuum, optionally the internal volume is substantially sealed from both open ends (5, 8) when vacuum is applied through the proximal open end (5) and tissue is drawn into the internal volume through the distal open end (8).

11. The add-on cap according to any of the preceding claims, wherein a proximal end of the add-on cap fits an exterior surface of a distal end of the instrument.

12. The add-on cap according to any of the preceding claims, wherein the at least one closed sidewall (2) is a substantially cylindrical closed surface.

13. A system for electroporation of tissue comprising:
- an endoscope;
- an add-on cap (1) according to any of the preceding claims, the add-on cap adapted to be mounted on the tip of the endoscope and extending in an axial direction of the endoscope; and
- an electric current generator,
- optionally the endoscope is a gastroscope, or a colonoscope, or a cystoscope, or a nephroscope, or a bronchoscope, a sigmoideumscope or a borescope.

14. The system according to claim 13, further comprising a vacuum generator in connection with the internal volume through the proximal open end (5) of the add-on cap.

15. The system according to claim 13 or 14, further comprising means for measuring an impedance between the two electrodes (3) of the add-on cap.

16. The system according to any of claims 13 to 15, further comprising processing means configured to compare a temperature measured by the temperature sensor (4) against a predetermined temperature threshold; and/or configured to compare an impedance between the two electrodes (3) on opposite sides of the internal volume against a predetermined impedance threshold.

## Patentansprüche

1. Zusatzkappe (1) für Elektroporation von Gewebe, die Folgendes umfasst:
- mindestens eine geschlossene Seitenwand (2), wie zum Beispiel eine zylindrische Seitenwand; ein proximales offenes Ende (5); ein distales offenes Ende (8); wobei die mindestens eine geschlossene Seitenwand und das proximale und distale offene Ende einen Hohlkörper definieren, der ein Innenvolumen aufweist;
- zwei Elektroden (3) auf gegenüberliegenden Seiten des Innenvolumens, wobei die Elektroden zur Anlegung eines elektrischen Felds für Elektroporation von Gewebe innerhalb des Innenvolumens konfiguriert sind,
**dadurch gekennzeichnet, dass** das proximale offene Ende (5) geeignet ist, um abdichtend in eine Spitze eines Instruments, wie zum Beispiel eines Endoskops, einzugreifen, und dass die mindestens eine geschlossene Seitenwand (2) und die offenen Enden eine gemeinsame Aufnahme für das Instrument und ein Innenvolumen für Elektroporation von Gewebe bilden.

2. Zusatzkappe nach Anspruch 1, wobei die Zusatzkappe geeignet ist, um auf die Spitze eines länglichen Instruments, wie zum Beispiel eines Endoskops, aufgebracht zu werden, wobei sich die Zusatzkappe in einer axialen Richtung des Instruments erstreckt.

3. Zusatzkappe nach Anspruch 2, wobei eine Längsachse der Zusatzkappe eine Verlängerung einer Längsachse des Instruments ist.

4. Zusatzkappe nach Anspruch 2 oder 3, wobei die Zusatzkappe eine direkte Sichtbarkeit von dem proximalen offenen Ende (5) zu dem distalen offenen Ende (8) bereitstellt.

5. Zusatzkappe nach einem der Ansprüche 2 bis 4, wobei das distale offene Ende (8) eine Öffnung ist, die im Wesentlichen senkrecht zu einer axialen Verlängerung des Instruments vorliegt.

6. Zusatzkappe nach einem der vorstehenden Ansprüche, wobei die zwei Elektroden (3) zur Messung einer Impedanz zwischen den zwei Elektroden konfiguriert sind.

7. Zusatzkappe nach einem der vorstehenden Ansprüche, wobei die zwei Elektroden (3) planare Elektroden sind, die auf der Innenseite der mindestens einen geschlossenen Seitenwand (2) aufgebracht sind.

8. Zusatzkappe nach einem der vorstehenden Ansprüche, die weiter mindestens einen Temperatursensor (4) umfasst, wobei optional der mindestens eine Temperatursensor (4) auf der Innenseite der mindestens einen geschlossenen Seitenwand zwischen den zwei Elektroden aufgebracht ist, optional zwei Temperatursensoren (4) auf gegenüberliegenden Seiten des Innenvolumens zwischen den zwei Elektroden aufgebracht sind.

9. Zusatzkappe nach einem der vorstehenden Ansprüche, die weiter Kabel (7) umfasst, die mit den zwei Elektroden (3) verbunden sind, wobei optional die Kabel (7) außerhalb der geschlossenen Seitenwand aufgebracht sind und mit den Elektroden durch Löcher in der geschlossenen Seitenwand verbunden sind.

10. Zusatzkappe nach einem der vorstehenden Ansprüche, wobei das proximale offene Ende (5) abdichtend in das Instrument eingreift, sodass Vakuum an das Innenvolumen durch das proximale offene Ende angelegt werden kann, optional mit Elektroporation zu behandelndes Gewebe in das Innenvolumen durch das distale offene Ende (8) durch das erzeugte Vakuum gezogen wird, optional das Innenvolumen im Wesentlichen von beiden offenen Enden (5, 8) abgedichtet ist, wenn durch das proximale offene Ende (5) Vakuum angelegt wird und Gewebe in das Innenvolumen durch das distale offene Ende (8) gezogen wird.

11. Zusatzkappe nach einem der vorstehenden Ansprüche, wobei ein proximales Ende der Zusatzkappe auf eine Außenoberfläche eines distalen Endes des Instruments passt.

12. Zusatzkappe nach einem der vorstehenden Ansprüche, wobei die mindestens eine geschlossene Seitenwand (2) eine im Wesentlichen zylindrische geschlossene Oberfläche ist.

13. System für Elektroporation von Gewebe, das Folgendes umfasst:
- ein Endoskop;
- eine Zusatzkappe (1) nach einem der vorstehenden Ansprüche, wobei die Zusatzkappe geeignet ist, um auf die Spitze des Endoskops aufgebracht zu werden und sich in einer axialen Richtung des Endoskops zu erstrecken; und
- einen Generator für elektrischen Strom,
- wobei optional das Endoskop Folgendes ist: ein Gastroskop oder ein Koloskop oder ein Zystoskop oder ein Nephroskop oder ein Bronchoskop, ein Sigmoideoskop oder ein Boreskop.

14. System nach Anspruch 13, das weiter einen Vakuumerzeuger in Verbindung mit dem Innenvolumen durch das proximale offene Ende (5) der Zusatzkappe umfasst.

15. System nach Anspruch 13 oder 14, das weiter Mittel für die Messung einer Impedanz zwischen den zwei Elektroden (3) der Zusatzkappe umfasst.

16. System nach einem der Ansprüche 13 bis 15, das weiter Verarbeitungsmittel umfasst, die zum Vergleich einer Temperatur, die durch den Temperatursensor (4) gemessen wird, mit einer vorgegebenen Temperaturschwelle konfiguriert sind und/oder zum Vergleich einer Impedanz zwischen den zwei Elektroden (3) auf gegenüberliegenden Seiten des Innenvolumens mit einer vorgegebenen Impedanzschwelle konfiguriert sind.

## Revendications

1. Capuchon complémentaire (1) destiné à l'électroporation d'un tissu comprenant :
- au moins une paroi latérale fermée (2), telle qu'une paroi latérale cylindrique ; une extrémité ouverte proximale (5) ; une extrémité ouverte distale (8) ; l'au moins une paroi latérale fermée et les extrémités ouvertes proximale et distale définissant un corps creux présentant un volume interne ;
- deux électrodes (3) sur les côtés opposés du volume interne ; les électrodes configurées pour appliquer un champ électrique pour l'électroporation d'un tissu à l'intérieur du volume interne,
**caractérisé en ce que** l'extrémité ouverte proximale (5) est conçue pour entrer en prise de manière étanche avec une pointe d'un instrument, tel qu'un endoscope et **en ce que** l'au moins une paroi latérale fermée (2) et les extrémités ouvertes forment un récepteur combiné pour l'instrument et le volume interne pour l'électroporation d'un tissu.

2. Capuchon complémentaire selon la revendication 1, dans lequel le capuchon complémentaire est conçu pour être monté sur la pointe d'un instrument allongé, tel qu'un endoscope, le capuchon complémentaire s'étendant dans une direction axiale de l'instrument.

3. Capuchon complémentaire selon la revendication 2, dans lequel un axe longitudinal du capuchon complémentaire est une extension d'un axe longitudinal de l'instrument.

4. Capuchon complémentaire selon la revendication 2 ou la revendication 3, dans lequel le capuchon complémentaire fournit une visibilité directe de l'extrémité ouverte proximale (5) à l'extrémité ouverte distale (8).

5. Capuchon complémentaire selon l'une quelconque des revendications 2 à 4, dans lequel l'extrémité ouverte distale (8) est une ouverture substantiellement perpendiculaire à une extension axiale de l'instrument.

6. Capuchon complémentaire selon l'une quelconque des revendications précédentes, dans lequel les deux électrodes (3) sont configurées pour mesurer une impédance entre les deux électrodes.

7. Capuchon complémentaire selon l'une quelconque des revendications précédentes, dans lequel les deux électrodes (3) sont des électrodes planes montées à l'intérieur de l'au moins une paroi latérale fermée (2).

8. Capuchon complémentaire selon l'une quelconque des revendications précédentes, comprenant en outre au moins un capteur de température (4), facultativement l'au moins un capteur de température (4) est monté sur l'intérieur de l'au moins une paroi latérale fermée entre les deux électrodes, facultativement deux capteurs de température (4) sont montés sur des côtés opposés du volume interne entre les deux électrodes.

9. Capuchon complémentaire selon l'une quelconque des revendications précédentes, comprenant en outre des câbles (7) connectés aux deux électrodes (3), facultativement les câbles (7) sont montés à l'extérieur de la paroi latérale fermée et se connectent aux électrodes à travers des trous dans la paroi latérale fermée.

10. Capuchon complémentaire selon l'une quelconque des revendications précédentes, dans lequel l'extrémité ouverte proximale (5) est entrée en prise de manière étanche avec l'instrument de sorte qu'un vide peut être appliqué dans le volume interne à travers l'extrémité ouverte proximale, facultativement le tissu devant être traité à l'électroporation est aspiré jusque dans le volume interne à travers l'extrémité ouverte distale (8) par le vide généré, facultativement le volume interne est substantiellement isolé de manière étanche des deux extrémités ouvertes (5, 8) quand le vide est appliqué à travers l'extrémité ouverte proximale (5) et le tissu est aspiré jusque dans le volume interne à travers l'extrémité ouverte distale (8).

11. Capuchon complémentaire selon l'une quelconque des revendications précédentes, dans lequel une extrémité proximale du capuchon complémentaire s'ajuste avec une surface extérieure d'une extrémité distale de l'instrument.

12. Capuchon complémentaire selon l'une quelconque des revendications précédentes, dans lequel l'au moins une paroi latérale fermée (2) est une surface fermée substantiellement cylindrique.

13. Système pour l'électroporation d'un tissu comprenant :
- un endoscope :
- un capuchon complémentaire (1) selon l'une quelconque des revendications précédentes, le capuchon complémentaire conçu pour être monté sur la pointe de l'endoscope et s'étendant dans une direction axiale de l'endoscope ; et
- un générateur de courant électrique,
- facultativement l'endoscope est un gastroscope, ou un coloscope, ou un cystoscope, ou un néphroscope, ou un bronchoscope, ou un sigmoïdoscope ou un horoscope.

14. Système selon la revendication 13, comprenant en outre un générateur de vide en connexion avec le volume interne à travers l'extrémité ouverte proximale (5) du capuchon complémentaire.

15. Système selon la revendication 13 ou 14, comprenant en outre un moyen de mesure d'une impédance entre les deux électrodes (3) sur le capuchon complémentaire.

16. Système selon l'une quelconque des revendications 13 à 15, comprenant en outre un moyen de traitement configuré pour comparer une température mesurée par le capteur de température (4) avec un seuil de température prédéterminé ; et/ou configuré pour comparer une impédance entre les deux électrodes (3) sur des côtés opposés du volume interne avec un seuil d'impédance prédéterminé.
